(19) Europäisches Patentamt
European Patent Office
Office européen des brevets

(11) Veröffentlichungsnummer: **0 153 277**
**B1**

# (12) EUROPÄISCHE PATENTSCHRIFT

(45) Veröffentlichungstag der Patentschrift: **26.08.87**

(21) Anmeldenummer: **85810049.8**

(22) Anmeldetag: **11.02.85**

(51) Int. Cl.⁴: **C 07 C 149/23,** C 07 D 207/16, C 07 D 277/06, A 61 K 31/22, A 61 K 31/40, A 61 K 31/425

## (54) Neue Pleuromutilinderivate, Verfahren zu ihrer Herstellung und ihre Verwendung.

(30) Priorität: **17.02.84 DE 3405632**
**12.04.84 DE 3413708**

(43) Veröffentlichungstag der Anmeldung: **28.08.85 Patentblatt 85/35**

(45) Bekanntmachung des Hinweises auf die Patenterteilung: **26.08.87 Patentblatt 87/35**

(84) Benannte Vertragsstaaten: **AT BE CH DE FR GB IT LI LU NL SE**

(56) Entgegenhaltungen:
**FR - A - 2 526 019**

**BIOCHEMISTRY, Band 20, 1981, Seiten 546-552, American Chemical Society, Columbus, Ohio, US; G. HÖGENAUER u.a.: "Affinity labeling of Escherichia coli ribosomes with a covalently binding derivative of the antibiotic pleuromutilin"**

(73) Patentinhaber: **SANDOZ AG, Lichtstrasse 35, CH-4002 Basel (CH)**
(84) Benannte Vertragsstaaten: **BE CH FR GB IT LI LU NL SE**

(73) Patentinhaber: **SANDOZ-PATENT-GMBH, Humboldtstrasse 3, D-7850 Lörrach (DE)**
(84) Benannte Vertragsstaaten: **DE**

(73) Patentinhaber: **SANDOZ-ERFINDUNGEN Verwaltungsgesellschaft m.b.H., Brunner Strasse 59, A-1235 Wien (AT)**
(84) Benannte Vertragsstaaten: **AT**

(72) Erfinder: **Berner, Heinz, Geyergasse 2A, A-1180 Wien (AT)**
Erfinder: **Vyplel, Hermann, Quellenstrasse 65/7, A-1100 Wien (AT)**

**EP 0 153 277 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft Derivate von 14-O-[(1--Amino-2-methylpropan-2-yl)thioacetyl]mutilin und -19,20-dihydromutilin, insbesondere N-Acyl-14-O-[(1-amino-2-methylpropan-2-yl)thioacetyl]mutilin oder -19,20-dihydromutilin.

Insbesondere betrifft die Erfindung neue Pleuromutilinderivate der Formel

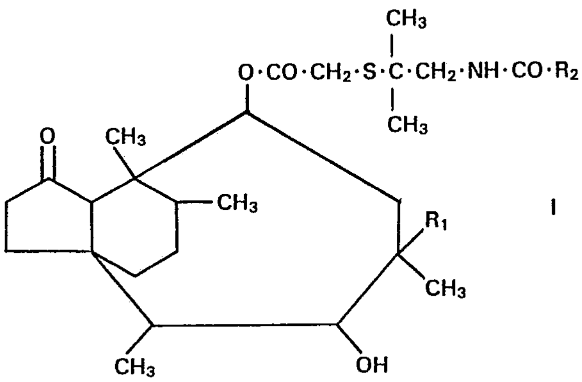

I

worin $R_1$ für Ethyl oder Vinyl und $R_2$ für eine Aminoalkylgruppe, deren Alkylteil durch Hydroxy substituiert sein kann, oder für einen fünfgliedrigen gesättigten Heterocyclus stehen, und ihre Säureadditions- und Quartärsalze, sowie Verfahren zu ihrer Herstellung und ihre Verwendung.

Erfindungsgemäss gelangt man zu den Verbindungen der Formel I, indem man eine Verbindungen der Formel

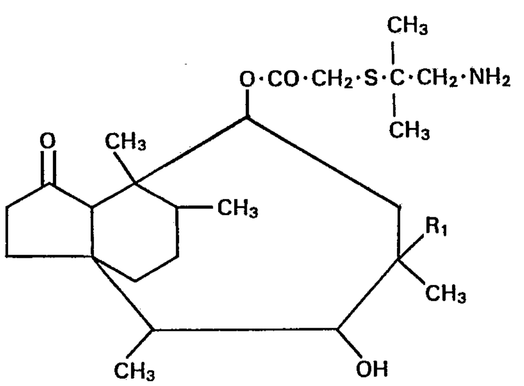

II

worin $R_1$ obige Bedeutung besitzt, acyliert, insbesondere mit einem Aktivester einer Verbindung der Formel

$HOOC\text{-}R_2^I$ III

worin $R_2^I$ die gleiche Bedeutung wie $R_2$ besitzt, wobei jedoch die in $R_2$ enthaltenen Aminogruppen durch eine Schutzgruppe geschützt sind, und die Schutzgruppe anschliessend abspaltet und die erhaltenen Verbindungen gewünschtenfalls in ihre Säureadditionssalze oder Quartärsalze überführt.

Das erfindungsgemässe Verfahren kann beispielsweise durchgeführt werden, indem man eine Verbindung der Formel II und eine Verbindung der Formel III in einem unter den Reaktionsbedingungen inerten Lösungsmittel, z.B. in einem Di(nieder)alkylcarbonsäureamid wie Dimethylformamid, löst oder suspendiert und die Reaktion bei Raumtemperatur oder erhöhter Temperatur, vorzugsweise bei Raumtemperatur, ablaufen lässt. Die anschliessende Abspaltung der Schutzgruppen kann nach an sich bekannten Methoden durchgeführt werden, beispielsweise durch reduktive Entschützung mit Pd/Aktivkohle und Wasserstoff oder durch Behandeln mit Trifluoressigsäure. Aus dem Reaktionsgemisch kann nach an sich bekannten Methoden das Endprodukt isoliert und gegebenenfalls gereinigt werden.

Die Verbindungen der Formel I können in ihre Säureadditionssalze überführt werden und umgekehrt. Aus den Verbindungen der Formel I können nach an sich bekannten Methoden die entsprechenden Quartärsalze erhalten werden.

Die Verbindungen der Formel I können zumindest ein asymmetrisches Kohlenstoffatom besitzen und daher in Form diastereoisomerer Isomere und derer Gemische vorliegen, die nach bekannten Methoden getrennt werden können. Die Verwendung von optisch aktiven Ausgangsmaterialien führt zu den entsprechenden Endprodukten. Die Erfindung betrifft sowohl die Isomeren wie auch deren Gemische.

Die Verbindungen der Formel I und ihre pharmakologisch veträglichen Säureadditions- und Quartärsalze besitzen bei geringer Toxizität interessante biologische, insbesondere chemotherapeutische Eigenschaften und können daher als Heilmittel verwendet werden. Sie entfalten eine Hemmwirkung gegen Bakterien, wie sie durch Untersuchungen in vitro mit dem Reihenverdünnungstest unter Verwendung verschiedener Bakterienstämme, z.B. Staph. aureus, Staph. epidermidis, Strept. pyogenes, Strept. pneumoniae, E. coli, Klebsiella pneumoniae, Haemophilus spp., Leptospiren spp., Erysipelothrix rhusiopathiae und obligate Anaerobier, z.B. Bacteroides fragilis, ab einer Konzentration von ca. 0,008 bis 25 µg/ml festgestellt wurde. Insbesondere wurde auch eine Hemmwirkung gegen Mykoplasmen und Chlamydien gefunden, die sich ab einer Konzentration von ca. 0,008 bis 0,5 µg/ml zeigte. Die chemotherapeutische Aktivität der Verbindungen wurde durch Versuche an Mäusen, unter Verwendung verschiedener Bakterienstämme, und bei Hühnern, unter Verwendung von Mykoplasmastämmen, nachgewiesen. Diese Hemmwirkung wurde ab einer Konzentration von ca. 12 bis 50 mg/kg Körpergewicht beobachtet. Daher können die erfindungsgemässen Verbindungen als antibakteriell wirksame Antibiotika verwendet werden.

Zusätzlich entfalten die oben genannten Verbindungen eine antiparasitäre Wirksamkeit, insbesondere gegen Kokzidien, sowie eine wachstumsfördernde Aktivität («Growth promotion»). Zur Feststellung der Wirksamkeit gegen Kokzidien erfolgte

die Prüfung in vivo am Huhn. Die Wirkungen lassen sich in diesen Tierversuchen in Dosierungen von 20-150 mg/kg Futter, in Abhängigkeit von der Applikationsdauer, bestätigen. Die wachstumsfördernden Eigenschaften werden bei Huhn und Schwein im Dosierbereich von 10-50 mg/kg Futter ermittelt. Die Verbindungen der Formel I erscheinen daher zur chemotherapeutischen Behandlung von Kokzidiosen beim Geflügel sowie als «Growth promoter» bei den genannten Tierarten geeignet.

Die Verbindungen der Formel I können oral, lokal oder parenteral verabreicht werden. Bei der Herstellung der entsprechenden Verabreichungsformen können die Verbindungen der Formel I gegebenenfalls als wasserlösliche, physiologisch verträgliche Salze allein oder in geeigneten Arzneiformen gemeinsam mit anorganischen oder organischen, pharmakologisch indifferenten Hilfsstoffen verabreicht werden. Beispielsweise werden sie als Bestandteile von Kapseln, Tabletten, Granulaten, gelatinüberzogenen Pulvern, Injektions- und Instillationszubereitungen eingesetzt, die eine zur Erreichung eines optimalen Butspiegels ausreichende Menge aktiver Verbindungen enthalten, das sind beispielsweise ca. 50-500 mg pro Kapsel. Überdies bilden die erfindungsgemässen Verbindungen hervorragende Zusätze zu Futtermittelmischungen (als Prämix) oder zum Trinkwasser sowie zur Verdünnerflüssigkeiten für die künstliche Besamung und für Eggdipping-Techniken.

Für die Anwendung hängt die zu verabreichende Dosis von der verwendeten Verbindung und der Verabreichungsart sowie der Behandlungsart ab. Man erhält im allgemeinen als antibakterielle und antianaerobe Mittel zufriedenstellende Ergebnisse, wenn die Verbindungen in einer täglichen Dosis von etwa 10 bis 300 mg/kg Körpergewicht eingesetzt werden. Bei grösseren Säugetieren werden zufriedenstellende Ergebnisse bei Verabreichung einer täglichen Dosis von ca. 1 bis 3 g erhalten. Diese Menge kann gegebenenfalls in entsprechend kleineren Dosen zwei- bis viermal täglich oder in Retardform gegeben werden. Die Verbindungen der Formel I können in Form der freien Basen oder in Form pharmazeutisch unbedenklicher Säureadditionssalze verwendet werden, wobei die Salze grössenordnungsmässig die gleiche Wirksamkeit besitzen wie die entsprechenden freien Basen. Geeignete Säureadditionssalze sind die Hydrochloride, Hydrogenfumarate, Fumarate und Naphthalin-1,5-sulfonate.

Als indifferente Hilfs- oder Zusatzstoffe zur Herstellung entsprechender galenischer Verabreichungsformen lassen sich Süssstoffe, Aromen, Farbstoffe, Konservierungsmittel, Füll- bzw. Trägerstoffe, beispielsweise Verdünnungsmittel, wie Kalziumkarbonat, Natriumkarbonat, Lactose, Polyvinylpyrrolidon, Mannit oder Talkum, Granulier- und Zerfallhilfsmittel, wie Stärke oder Alginsäure, Bindemittel, wie Stärke, Gelatine oder Akazie und Gleitmittel, wie Magnesiumstearat, Stearinsäure oder Talkum, verwenden.

Oral verabreichbare Zubereitungen können übliche Suspendiermittel enthalten, beispielsweise Methylcellulose, Tragacanth oder Natriumalginat. Als Netzmittel eignen sich beispielsweise Lecithin, Polyoxyethylenstearat oder Polyoxyethylensorbitanmonooleat. Als Konservierungsmittel lässt sich beispielsweise Ethyl-o-hydroxybenzoat verwenden. Zur Herstellung von Kapseln kann man als Verdünnungsmittel beispielsweise Kalziumkarbonat, Kalziumphosphat oder Kaolin einsetzen.

Die Verbindungen der Formel I können in ähnlicher Weise wie die für diese Verwendung bekannte Standardverbindung Tiamulin eingesetzt werden.

Die Ausgangsverbindungen der Formel II sind neu und können erhalten werden, indem man eine Verbindung der Formel

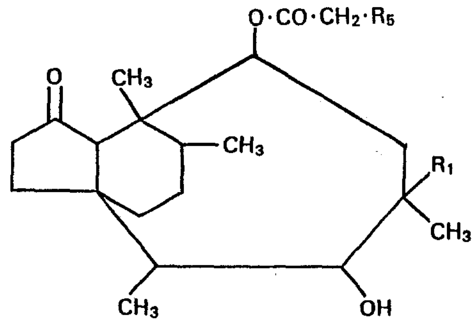

IV

worin $R_1$ obige Bedeutung besitzt und $R_5$ für Chlor, Brom oder eine $-O\cdot SO_2\cdot R_6$-Gruppe, wobei $R_6$ Alkyl oder Aryl bedeutet, steht, mit der Verbindung der Formel

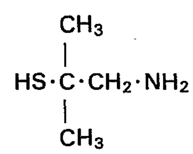

V

umsetzt. Diese Umsetzung kann beispielsweise ausgeführt werden, indem man in einer Lösung von Natrium in einem wasserfreien niederen Alkohol, z.B. in Ethanol oder Methanol, eine Verbindung der Formel V löst. Zu dieser Lösung wird nun die Lösung einer Verbindung der Formel IV in einem unter den Reaktionsbedingungen inerten Lösungsmittel, z.B. in einem aliphatischen Keton, wie Ethylmethylketon oder Aceton, zugesetzt. Die Reaktion verläuft vorzugsweise bei Raumtemperatur bis zur Siedetemperatur des Reaktionsgemisches, insbesondere bei 25 bis 55°C, und dauert beispielsweise zwischen 7 und 15 Stunden.

Die Ausgangsprodukte der Formeln III, IV und V sind bekannt oder analog zu bekannten Methoden bzw. wie in den Beispielen beschrieben herstellbar. Die Verbindungen der Formel V sind beispielsweise wie in F. J. Carroll, J. D. White und M. E. Wall, J. Org. Chem. 28/1240 (1963) beschrieben erhältlich.

Die als Substituenten aufscheinenden Alkylgruppen bedeuten vorzugsweise nieder Alkylgruppen, insbesondere mit 1 bis 4 Kohlenstoffatomen. Steht $R_2$ für einen Heterocyclus, so kann dieser ein oder zwei Heteroatome enthalten, wobei ein Heteroatom Stickstoff und das gegebenenfalls vorhandene zweite Heteroatom Schwefel ist.

Als Schutzgruppen für die Aminofunktion in den Ausgangsverbindungen der Formel III können die allgemein als Aminoschutzgruppen bekannten eingesetzt werden, beispielsweise $-CO \cdot O \cdot CH_2 \cdot C_6H_5$, $-CO \cdot O \cdot C(CH_3)_3$ oder $-CO \cdot O \cdot CH_2 \cdot CCl_3$.

Bevorzugte Bedeutungen der Substituenten sind folgende:

$R_1$ = a) Ethyl
  b) Vinyl, wobei Vinyl besonders bevorzugt ist

$R_2$ = a) Aminoalkyl
  b) Aminohydroxyalkyl
  c) wie a) und b), wobei der Alkylteil 1 bis 6, vorzugsweise 1 bis 4 Kohlenstoffatome besitzt
  d) 5-gliedriger gesättigter Heterocyclus
  e) 5-gliedriger gesättigter Heretocyclus, der ein Stickstoffatom und gegebenenfalls ein Schwefelatom enthält.

Kombinationen dieser Gruppen sind besonders bevorzugt.

Eine besonders bevorzugte Verbindung ist das 14-O-[1-(2-Amino-3-methylbutyrylamino)-2-methylpropan-2-ylthioacetyl]mutilin als freie Base oder als Hydrochlorid, insbesondere in der D-Form.

In den nachfolgenden Beispielen, die die Erfindung näher erläutern, ihren Umfang aber in keiner Weise einschränken sollen, erfolgen alle Temperaturangaben in Celsiusgraden.

*Beispiel 1*

*14-O-[1-(2-Amino-3-methylbutyrylamino)-2-methylpropan-2-yl-thioacetyl]-19,20-dihydromutilin · Hydrochlorid:*

Eine Lösung von 1,85 g Z-Valin-4-nitrophenylester und 2,35 g 14-O-[(1-Amino-2-methylpropan-2-yl)-thioacetyl]-19,20-dihydromutilin in 30 ml Dimethylformamid wird 7 Stunden bei 25° gehalten. Das Reaktionsgemisch wird anschliessend auf Wasser gegossen und wiederholt mit Essigsäureethylester ausgeschüttelt. Nach Rückwaschen der organischen Phase mit 0,1 N Salzsäure und anschliessend mit NaCl-gesättigtem Wasser erhält man die geschützte Titelverbindung, die ohne weitere Reinigung zur Entschützung eingesetzt wird.

Eine Lösung von 3,1 g dieser Z-geschützten Verbindung in 150 ml Ethanol wird mit 60 mg Pd/Aktivkohle (Palladium auf Aktivkohle, 10%ig) versetzt und unter Wasserstoffatmosphäre 1 Stunde gerührt. Man erhält in praktisch quantitativer Ausbeute die Titelverbindung.

Das Verfahren kann auch mit BOC-Valin-4-nitrophenylester durchgeführt werden, wobei die Entschützung folgendermassen ausgeführt wird:

3 mMol der BOC-geschützten Verbindung werden bei −10° in 25 ml Trifluoressigsäure gelöst und bei dieser Temperatur 10 Minuten gehalten. Anschliessend bringt man das Reaktionsgemisch auf 25°, lässt weitere 2 Stunden reagieren und giesst in der Folge auf 100 ml 10%ige $NaHCO_3$-Lösung. Nach wiederholtem Extrahieren mit Essigsäureethylester und Rückwaschen der organischen Phase erhält man das Rohprodukt, das über Kieselgel (Laufmittel: $CHCl_3/CH_3OH$ = 7/l) chromatographiert wird.

Analog wie in Beispiel 1 beschrieben, können auch folgende Verbindungen der Formel I erahlten werden:

| Bsp: | $R_1$ | $R_2$ |
|---|---|---|
| 2 | $-CH=CH_2$ | $-CH_2 \cdot NH_2$ |
| 3 | — » — | $-CH \cdot CH_3$ (D)<br>\|<br>$NH_2$ |
| 4 | — » — | $-CH \cdot CH_2 \cdot CH \cdot CH_3$<br>\| \|<br>$NH_2$ $CH_3$ |
| 5 | $-C_2H_5$ | $-CH \cdot CH_3$ (D)<br>\|<br>$NH_2$ |
| 6 | $-CH=CH_2$ | $-CH \cdot CH_3$ (L)<br>\|<br>$NH_2$ |
| 7 | — » — | 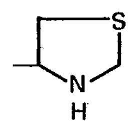 (L) |
| 8 | — » — | 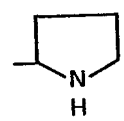 (L) |
| 9 | $-C_2H_5$ | $-CH_2CH_2 \cdot NH_2$ |
| 10 | $-CH=CH_2$ | $-CH - CH-CH_3$<br>\| \|<br>$NH_2$ $CH_3$ (L) |
| 11 | — » — | 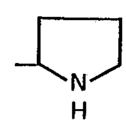 (D) |
| 12 | — » — | $-CH - CH - CH_3$<br>\| \|<br>$NH_2$ $CH_3$ (D) |
| 13 | — » — | $-CH - CH_2OH$<br>\|<br>$NH_2$ (L) |

*NMR-Spektren ($CDCl_3$)*

| *Beispiel:* | *Spektrum:* |
|---|---|
| 1 | 5,62 (d, 1H, $H_{14}$, $J_{H14H13}$ = 7,5 Hz); 7,72 (t, 1H, NHCO); 3,42 (d, 1H, N-CH-CO, J = 6,25 Hz); 3,32 (d, 1H, $H_{11}$, $J_{H11H10}$ = 6,25 Hz); AB-System: ($V_A$ = 3,17, $V_B$ = 3,27, $J_{AB}$ = 15 Hz, $S-CH_2-CO$). |

| Beispiel: | Spektrum: |
|---|---|
| 2 | 7,68 (t, 1H, NH); 5,73 (d, 1H, $H_{14}$, $J_{H14H13}$ = 8,75 Hz); 3,75 (s, 2H, CO-$CH_2$-$NH_2$); 3,38 (d, 1H, $H_{11}$, $J_{H11H10}$ = 6,25 Hz); 3,28 (m, 2H, ≡C-$CH_2$-NH); 1,25, 1,26 (s, s, 6H, gem. $CH_3$). |
| 3 | 7,78 (t, 1H, NH); 5,74 (d, 1H, $H_{14}$, $J_{H14H13}$ = 8,75 Hz); 4,03 (q, 1H, -CH-$CH_3$, J = 7,5 Hz); 3,37 (d, 1H, $H_{11}$, $J_{H11H10}$ = 6,25 Hz); 1,25 (s, 2×$CH_3$, gem. $CH_3$); 3,3 (m, 2H, ≡ C-$CH_2$-NH). |
| 4 | 7,76 (t, 1H, NH); 5,76 (d, 1H, $H_{14}$, $J_{H14H13}$ = 8,75 Hz); 3,43 (dd, 1H, CO-$CH_2$ <br> \| <br> CH-$NH_2$, J = 3,75 Hz, J' = 8,75 Hz); 3,25 (d, 2H, CH-$CH_2$-$NH_2$); AB-System: ($V_A$ = 3,17, $V_B$ = 3,25, $J_{AB}$ = 15 Hz, -S-$CH_2$CO). |
| 5 | 5,64 (d, 1H, $H_{14}$, $J_{H14H13}$ = 8,1 Hz); 3,56 (q, 1H, $CH_3$-CH, J = 7,5 Hz); 3,44 (d, 1H, $H_{11}$, $J_{H11H10}$ = 6,25 Hz); AB-System: ($V_A$ = 3,17, $V_B$ = 3,25, $J_{AB}$ = 15 Hz, S-$CH_2$-CO); ABX-System: ($V_A$ = 3,33, $V_B$ = 3,25, $V_X$ = 7,75, $J_{AB}$ = 13,4 Hz, $J_{AX}$ = $J_{BX}$ = 7,5 Hz, C-$CH_2$-NH). |
| 6 | 7,74 (t, 1H, NH); 5,75 (d, 1H, $H_{14}$, $J_{H14H13}$ = 8,75 Hz); 3,56 (q, 1H, CO-$CH_3$ <br> \| <br> CH-NH, J = 7,5 Hz); 3,36 (d, 1H, $H_{11}$, $J_{H11H10}$ = 6,25 Hz); AB-System: ($V_A$ = 3,17, $V_B$ = 3,24, $J_{AB}$ = 15 Hz, S-$CH_2$-CO); 1,4 (d, 3H, CO-CH-$NH_2$ (with $CH_3$ above CH), J = 7,5 Hz). |
| 7 | 7,72 (t, 1H, NH); 5,77 (d, 1H, $H_{14}$, $J_{H14H13}$ = 8,75 Hz); AB-System: ($V_A$ = 4,29, $V_B$ = 4,13, $J_{AB}$ = 10 Hz, S-$CH_2$-NH); 3,36 (m, 1H, $H_{11}$). |
| 8 | 8,07 (m, 1H, NH); 5,76 (d, 1H, $H_{14}$, $J_{H14H13}$ = 8,75 Hz); 3,84 (dd, 1H, CO-$CH_2$ <br> \| <br> CH-NH, J = 5 Hz, J' = 8,75 Hz); 3,38 (d, 1H, $H_{11}$, $J_{H11H10}$ = 6,25 Hz); AB-System: ($V_A$ = 3,17, $V_B$ = 3,25, $J_{AB}$ = 15 Hz, S-$CH_2$-CO); 3,25 (m, 2H, ≡ C-$CH_2$-NH). |
| 9 | 5,62 (d, 1H, $H_{14}$, $J_{H14H13}$ = 7,5 Hz); 3,24 (d, 1H, $H_{11}$, $J_{H11H10}$ = 6,25 Hz); ABX-System: ($V_A$ = 3,34, $V_B$ = 3,26, $V_X$ = 7,38, $J_{AB}$ = 13,5 Hz, $J_{AX}$= $J_{BX}$ = 7,5 Hz, NH-$CH_2$-C ≡); 3,83 (t, 2H, $NH_2$-$CH_2$, J = 5 Hz); 2,38 (t, 2H, $CH_2$CO, J = 5 Hz). |
| 10 | 7,68 (m, 1H, NH); 5,74 (d, 1H, $H_{14}$, $J_{H14H13}$ = 8,75 Hz); 3,2 (s, 2H, S-$CH_2$-CO); 3,38 (d, 1H, $H_{11}$); 3,25 (d, 1H, CH-$CH_3$, J = 3,75 Hz). |
| 11 | 8,05 (t, 1H, NH, NH-$CH_2$-C≡); 5,73 (d, 1H, $H_{14}$, $J_{H14H13}$ = 8,75 Hz); 4,48 (dd, $CH_2$ <br> \| <br> 1H, NH-CH-CO, J = 8,75, J' = 6,25); 1,25, 1,22 (s, s, gem. $CH_3$); AB-System: ($V_A$ = 3,18, $V_B$ = 3,24, $J_{AB}$ = 15 Hz, S-$CH_2$-CO). |
| 12 | 7,8 (m, 1H, NH); 5,75 (d, 1H, $H_{14}$, $J_{H14H13}$ = 8,75 Hz); 3,38 (d, 1H, $H_{11}$, $J_{H11H10}$ = 6,25 Hz); 3,24 (d, 1H, CH-$NH_2$); 3,2 (s, 2H, S-$CH_2$-CO(; 3,31 (m, 2H, $CH_2$-NHCO). |
| 13 | 7,9 (m, 1H, NH); 5,75 (d, 1H, $H_{14}$, $J_{H14H13}$ = 8,75 Hz); AB-System: ($V_A$ = 3,18, $V_B$ = 3,28, $J_{AB}$ = 15 Hz, S-$CH_2$-CO); 3,36 (m, 1H, $H_{11}$); 3,91 (dd, 1H, -CH<u>H</u>'-OH, $J_1$ = 10 Hz, $J_2$ = 5 Hz); 3,71 (dd, 1H, -C<u>H</u>H'-OH, $J_1$ = 10 Hz, $J_2$ = 6,25 Hz); 3,5 (dd, 1H, $NH_2$-<u>CH</u>-$CH_2$, $J_1$ = 6,25 Hz, $J_2$ = 5 Hz). |

Das als Ausgangsprodukt benötigte 14-O-[(1-Amino-2-methylpropan-2-yl)thioacetyl]-19,20-dihydromutilin kann folgendermassen erhalten werden:

4,6 g Natrium werden in 500 ml Ethanol (absolut) gelöst, mit 14,1 g 3-Amino-2-methyl-2-propylmercaptan [F.I. Carroll, J.D. White und M.E. Wall, J. Org. Chem. 28, 1240 (1963)] versetzt und 1 Stunde bei 25° gerührt. Das Reaktionsgemisch wird anschliessend mit einer Lösung aus 53,2 g 19,20-Dihydropleuromutilin-22-O-tosylat in 300 ml Ethylmethylketon versetzt und 15 Stunden bei 25° gehalten. Man giesst in der Folge auf Eiswasser und extrahiert wiederholt mit Essigsäureethylester. Nach Rückwaschen der organischen Phase mit NaCl-gesättigtem Wasser erhält man ein Rohprodukt, das über Kieselgel (Laufmittel: $CHCl_3$/$CH_3OH$ = 7/1) chromatographiert wird.

NMR ($CDCl_3$): 5,8 (d, 1H, $H_{14}$, $H_{14}$, $J_{H14H13}$ = 9 Hz); 3,38 (d, 1H, $H_{11}$, $J_{H11H10}$ = 6,3 Hz); 3,17 (s, 2H, S-$CH_2$-CO); 2,65 (s, 2H, N-$CH_2$-CH≡); 1,7 (b, 2H, $NH_2$); 1,28 (s, 6H, 2 × $CH_3$).

**Patentansprüche für die Vertragsstaaten:**
BE, CH, FR, GB, IT, LI, LU, DE, NL, SE,

1. Ein N-Acyl-14-O-[(1-amino-2-methylpropan-2-yl)thioacetyl]mutilin oder 19,20-dihydromutilin.
2. Pleuromutilinderivate der Formel

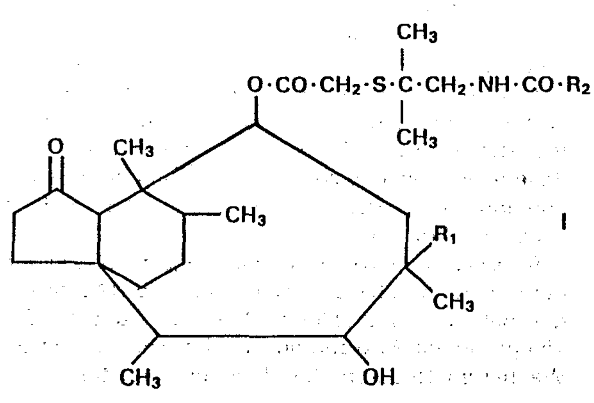

I

worin $R_1$ für Ethyl oder Vinyl und $R_2$ für eine Aminoalkylgruppe, deren Alkylteil durch Hydroxy substituiert sein kann, oder für einen fünfgliedrigen gesättigten Heterocyclus stehen, und ihre Säureadditions- und Quartärsalze.

3. Eine Verbindung entsprechend Anspruch 2, worin
a) $R_1$ Vinyl und $R_2$ ein fünfgliedriger gesättigter Heterocyclus oder eine unsubstituierte Aminoalkylgruppe bedeuten, und die, falls sie ein asymmetrisches Kohlenstoffatom enthält, in der D-Form vorliegt oder
b) $R_1$ Vinyl und $R_2$ Aminohydroxyalkyl bedeuten.

4. Eine Verbindung, ausgewählt von 14-O-[1-((D)-2-Amino-3-methylbutyrylamino)-2-methylpropan-2-yl-thioacetyl]-19,20-dihydromutilin, 14-O-[1-(Aminoacetylamino)-2-methylpropan-2-yl-thioacetyl]mutilin, 14-O-[1-((D)-2-Aminopropionylamino)-2-methylpropan-2-yl-thioacetyl]mutilin, 14-O-[1-(2-Amino-4-methylvalerylamino)-2-methylpropan-2-yl-thioacetyl]mutilin, 14-O-[1-((D)-2-Aminopropionylamino)-2-methylpropan-2-yl-thioacetyl]-19,20-dihydromutilin, 14-O-[1-((L)-2-Aminopropionylamino)-2-methylpropan-2-yl-thioacetyl]mutilin, 14-O-[1-((L)-Thiazolidin-4-yl-carbonylamino)-2-methylpropan-2-yl-thioacetyl]mutilin. 14-O-[1-((L)-Pyrrolidin-2-yl-carbonylamino)-2-methylpropan-2-yl-thioacetyl]mutilin, 14-O-[1-(3-Aminopropionylamino)-2-methylpropan-2-yl-thioacetyl]-19,20-dihydromutilin, 14-O-[1-((L)-2-Amino-3-methylbutyrylamino)-2-methylpropan-2-yl-thioacetyl]mutilin, 14-O-[1-((D)-Pyrrolidin-2-yl-carbonylamino)-2-methylpropan-2-yl-thioacetyl]mutilin und 14-O-[1-((L)-2-Amino-3-hydroxypropionylamino)-2-methylpropan-2-yl-thioacetyl]mutilin in Form der freien Basen, der Säureadditionssalze oder Quartärsalze.

5. 14-O-[1-((D)-2-Amino-3-methylbutyrylamino)-2-methylpropan-2-yl-thioacetyl]mutilin in Form der freien Base, der Säureadditionssalze oder Quartärsalze.

6. Eine chemotherapeutische Zusammensetzung, enthaltend eine Verbindung entsprechend einem der Ansprüche 1 bis 5 in Form der freien Base oder eines chemotherapeutisch akzeptablen Säureadditionssalzes oder Quartärsalzes, gemeinsam mit einem chemotherapeutisch akzeptablen Verdünnungs- oder Trägerstoff oder eine Futtermittel- oder Trinkwasserzusatzzusammensetzung, enthaltend eine Verbindung entsprechend einem der Ansprüche 1 bis 5 in Form der freien Base oder eines physiologisch annehmbaren Säureadditionssalzes oder Quartärsalzes, gemeinsam mit einem physiologisch annehmbaren Verdünnungs- oder Trägerstoff.

7. Eine Verbindung entsprechend einem der Ansprüche 1 bis 5, a) in Form der freien Base oder eines chemotherapeutisch akzeptablen Säureadditionssalzes oder Quartärsalzes, zur Verwendung als chemotherapeutisches Mittel oder b) in Form der freien Base oder eines physiologisch annehmbaren Säureadditionssalzes oder Quartärsalzes, zur Verwendung als Veterinärmittel.

8. Verwendung einer Verbindungs gemäss einem der Ansprüche 1 bis 5 zur Herstellung von Mitteln gegen Bakterien und obligate Anaerobier und zur Bekämpfung von Mikroorganismusinfektionen und zur Wachstumsförderung bei Haus- und Nutztieren.

9. Verfahren zur Herstellung von N-Acyl-14-O-[(1-amino-2-methylpropan-2-yl)thioacetyl]mutilin- und -19,20-dihydromutilin-Derivaten insbesondere die der Formel I wie im Anspruch 2 definiert, ihrer Säureadditionssalze und Quartärsalze, dadurch gekennzeichnet, dass man eine Verbindung der Formel

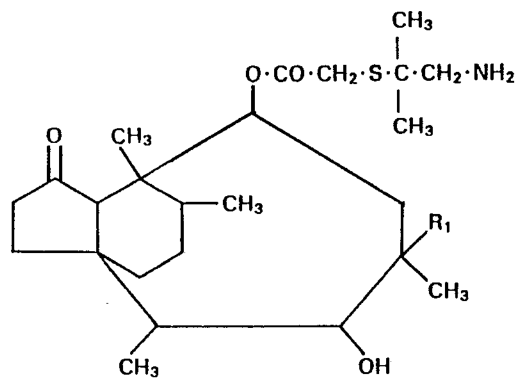

II

worin $R_1$ für Ethyl oder Vinyl steht, acyliert, insbesondere mit einem Aktivester der Verbindung der Formel

HOOC-$R_2^{I}$ III

worin $R_2^{I}$ die gleiche Bedeutung wie $R_2$ besitzt, wobei jedoch die in $R_2$ enthaltenen Aminogruppen durch eine Schutzgruppe geschützt sind, und die Schutzgruppe anschliessend abspaltet und die erhaltenen Verbindungen in freier Form oder Form ihrer Säureadditionssalze oder Quartärsalze zurückgewinnt.

10. Eine Verbindung der Formel II wie im Anspruch 9 definiert.

**Patentansprüche für den Vertragsstaat:** AT

1. Verfahren zur Herstellung von N-Acyl-14-O-[(1-amino-2-methylpropan-2-yl)thioacetyl]mutilin- und -19,20-dihydromutilin-Derivaten ihrer Säureadditionssalze und Quartärsalze, dadurch gekennzeichnet, dass man eine Verbindung Formel II

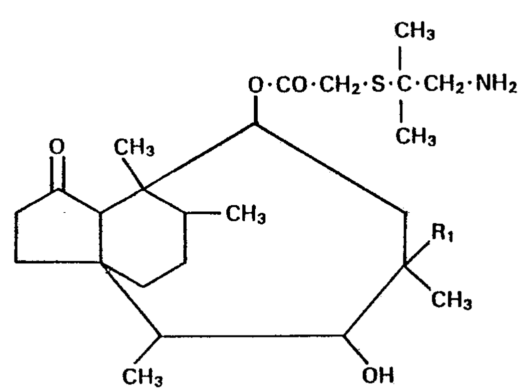

II

worin $R_1$ für Ethyl oder Vinyl steht, acyliert, und die erhaltenen Verbindungen in freier Form oder in Form ihrer Säureadditionssalze oder Quartärsalze zurückgewinnt.

2. Verfahren zur Herstellung von Pleuromutilinderivaten der Formel I

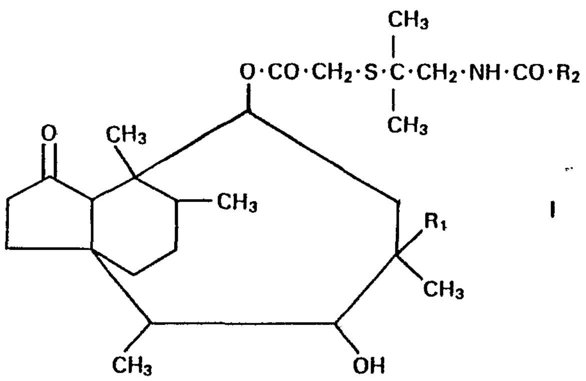

I

worin $R_1$ für Ethyl oder Vinyl und $R_2$ für eine Aminoalkylgruppe, deren Alkylteil durch Hydroxy substituiert sein kann, oder für einen fünfgliedrigen gesättigten Heterocyclus stehen, und ihrer Säureadditions- und Quartärsalze, dadurch gekennzeichnet, dass man eine Verbindung der Formel II

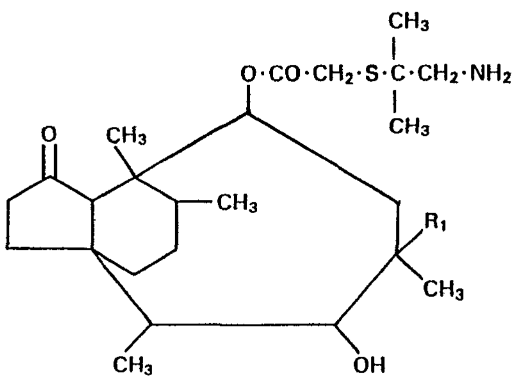

II

mit einem Aktivester der Verbindung der Formel III

$HOOC\text{-}R'_2$ III

acyliert,
worin $R'_2$ die gleiche Bedeutung wie $R_2$ besitzt, wobei jedoch die in $R_2$ enthaltenen Aminogruppen durch eine Schutzgruppe geschützt sind, und die Schutzgruppe anschliessend abspaltet und die erhaltenen Verbindungen in freier Form oder in Form ihrer Säureadditionssalze oder Quartärsalze zurückgewinnt.

3. Verfahren nach Anspruch 2, worin
a) $R_1$ Vinyl und $R_2$ ein fünfgliedriger gesättigter Heterocyclus oder eine unsubstituierte Aminoalkylgruppe bedeuten, und die, falls sie ein asymmetrisches Kohlenstoffatom enthält, in der D-Form vorliegt oder
b) $R_1$ Vinyl und $R_2$ Aminohydroxyalkyl bedeuten.

4. Verfahren nach Anspruch 2, worin die hergestellte Verbindung vom 14-O-[1-((D)-2-Amino-3-methylbutyrylamino)-2-methylpropan-2-yl-thioacetyl]-19,20-dihydromutilin, 14-O-[1-(Aminoacetylamino)-2-methylpropan-2-yl-thioacetyl]mutilin, 14-O-[1-((D)-2-Aminopropionylamino)-2-methylpropan-2-yl-thioacetyl]mutilin, 14-O-[1-(2-Amino-4-methylvalerylamino)-2-methylpropan-2-yl-thioacetyl]mutilin, 14-O-[1-((D)-2-Aminopropionylamino)-2-methylpropan-2-yl-thioacetyl]-19,20-dihydromutilin, 14-O-[1-((L)-2-Aminopropionylamino)-2-methylpropan-2-yl-thioacetyl]mutilin, 14-O-[1-((L)-Thiazolidin-4-yl-carbonylamino)-2-methylpropan-2-yl-thioacetyl]mutilin, 14-O-[1-((L)-Pyrrolidin-2-yl-carbonylamino)-2-methylpropan-2-yl-thioacetyl)mutilin, 14-O-[1-(3-Aminopropionylamino)-2-methylpropan-2-yl-thioacetyl]-19,20-dihydromutilin, 14-O-[1-((L)-2-Amino-3-methylbutyrylamino)-2-methylpropan-2-yl-thioacetyl]mutilin, 14-O-[1-((D)-Pyrrolidin-2-yl-carbonylamino)-2-methylpropan-2-yl-thioacetyl]mutilin, 14-O-[1-((L)-2-Amino-3-hydroxypropionylamino)-2-methylpropan-2-yl-thioacetyl]mutilin und 14-O-[1-((D)-2-Amino-3-methylbutyrylamino)-2-methylpropan-2-yl-thioacetyl]mutilin je in Form der freien Base, der Säureadditionssalze oder Quartärsalze ausgewählt wird.

5. Eine Verbindung ausgewählt von
a) einem N-Acyl-14-O-[(1-amino-2-methylpropan-2-yl)thioacetyl]-mutilin oder -19,20-dihydromutilin;
b) einer Verbindung der Formel I wobei die Substituenten wie im Anspruch 2 definiert sind;
c) einer Verbindung der Formel I worin die Substituenten wie im Anspruch 3 definiert sind;
d) einer der im Anspruch 4 angegebenen Verbindungen.

6. Eine Verbindung gemäss Anspruch 5 in Form der freien Base, der Säureadditionssalze oder Quartärsalze.

7. Eine chemotherapeutische Zusammensetzung, enthaltend eine Verbindung entsprechend Anspruch 5 in Form der freien Base oder eines chemotherapeutisch akzeptablen Säureadditionssalzes oder Quartärsalzes, gemeinsam mit einem chemotherapeutisch akzeptablen Verdünnungs- oder Trägerstoff oder eine Futtermittel- oder Trinkwasserzusatzzusammensetzung, enthaltend eine Verbindung entsprechend Anspruch 5 in Form der freien Base oder eines physiologisch annehmbaren Säureadditionssalzes oder Quartärsalzes, gemeinsam mit einem physiologisch annehmbaren Verdünnungs- oder Trägerstoff.

8. Eine Verbindung entsprechend Anspruch 5, a) in Form der freien Base oder eines chemotherapeutisch akzeptablen Säureadditionssalzes oder Quartärsalzes, zur Verwendung als chemotherapeutisches Mittel oder b) in Form der freien Base oder eines physiologisch annehmbaren Säureadditionssalzes oder Quartärsalzes, zur Verwendung als Veterinärmittel.

9. Verwendung einer Verbindung gemäss An-

spruch 5 zur Herstellung von Mitteln gegen Bakterien und obligate Anaerobier und zur Bekämpfung von Mikroorganismusinfektionen und zur Wachstumsförderung bei Haus- und Nutzieren.

10. Eine Zusammensetzung gemäss Anspruch 7 oder die Verwendung gemäss Anspruch 8 oder 9 worin die Verbindungen gemäss Anspruch 5, gemäss Anspruch 1 hergestellt werden.

**Claims for the Contracting States:**
BE, CH, DE, FR, GB, IT, LI, LU, NL, SE

1. An N-acyl-14-O-[(1-amino-2-methylpropan-2-yl)thioacetyl]-mutilin or 19,20-dihydromutilin.

2. Pleuromutilin derivatives of formula I

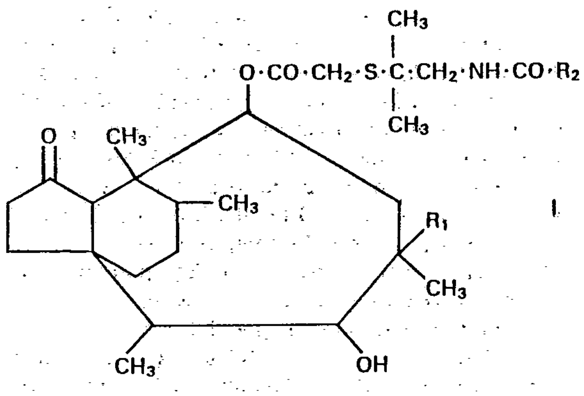

I

wherein $R_1$ stands for ethyl or vinyl and $R_2$ stands for an aminoalkyl group the alkyl moiety of which can be substituted by hydroxy or for a fivemembered saturated heterocycle
and their addition or quaternary salts.

3. A compound according to claim 2 wherein
a) $R_1$ represents vinyl and R represents a fivemembered saturated heterocycle or unsubstituted aminoalkyl and if containing an asymetric carbon atom is in (D)-Form or
b) $R_1$ represents vinyl and $R_2$ represents amino-hydroxyalkyl.

4. A compound selected from 14-O-[1-((D)-2-amino-3-methylbutyrylamino)-2-methylpropan-2-yl-thioacetyl]-19,20-dihydromutilin, 14-O-[1-(aminoacetylamino)-2-methylpropan-2-yl-thioacetyl]-mutilin, 14-O-[1-((D)-2-aminopropionylamino)-2-methylpropan-2-yl-thioacetyl]mutilin, 14-O-[1-(2-amino-4-methylvalerylamino)-2-methylpropan-2-yl-thioacetyl]mutilin, 14-O-[1-((D)-2-aminopropionylamino)-2-methylpropan-2-yl-thioacetyl]-19,20-dihydromutilin, 14-O-[1-((L)-2-aminopropionylamino)-2-methylpropan-2-yl-thioacetyl]mutilin, 14-O-[1-((L)-thiazolidin-4-yl-carbonylamino)-2-methylpropan-2-yl-thioacetyl]mutilin, 14-O-[1-((L)-pyrrolidin-2-yl-carbonylamino)-2-methylpropan-2-yl-thioacetyl]mutilin, 14-O-[1-(3-aminopropionylamino)-2-methylpropan-2-yl-thioacetyl]-19,20-dihydromutilin, 14-O-[1-((L)-2-amino-3-methylbutyrylamino)-2-methylpropan-2-yl-thioacetyl]mutilin, 14-O-[1-((D)-pyrrolidin-2-yl-carbonylamino)-2-methylpropan-2-yl-thioacetyl]mutilin and 14-O-[1-((L)-2-amino-3-hydroxypropionylamino)-2-methylpropan-2-yl-thioacetyl]mutilin in the form of the free base, the acid addition salts or the quaternary salts.

5. 14-O-[1-((D)-2-amino-3-methylbutyrylamino)-2-methylpropan-2-yl-thioacetyl]mutilin in the form of the free base, the acid addition salts or the quaternary salts.

6. A chemotherapeutic composition comprising a compound according to one of the claims 1 to 5 in the form of the free base or of a chemotherapeutically acceptable acid addition salt or quaternary salt together with a chemotherapeutically acceptable diluent or carrier or a feed or drinkwater additive composition comprising a compound according to one of the claims 1 to 5 in the form of the free base or of a physiologically acceptable acid addition salt or quaternary salt together with a physiologically acceptable diluent or carrier.

7. A compound according to one of the claims 1 to 5, a) in the form of the free base or of a chemotherapeutically acceptable acid addition salt or quaternary salt, for use as a chemotherapeutic agent or b) in the form of the free base, or of a physiologically acceptable acid addition salt or quaternary salt for use as a veterinary agent.

8. The use of a compound according to one of claims 1 to 5 for preparing agents against bacteria and obligatory anaerobes and for controlling microorganism infections and for promoting growth in domestic animals.

9. A process for preparing N-acyl-14-O-[1-amino-2-methylpropan-2-yl)thioacetyl]mutilin- and -19,20-dihydromutilin derivatives, in particular those of formula I as defined in claim 2, their acid addition salts and quaternary salts, characterised by acylating a compound of formula

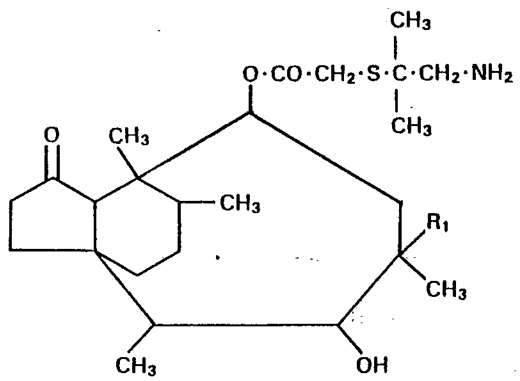

II

wherein $R_1$ stands for ethyl or vinyl in particular with an active ester of the compound of formula

$$HOOC\text{-}R_2^I \qquad III$$

wherein $R_2^I$ has the same meaning as $R_2$ whereby howerer the amino groups contained in $R_2$ are protected by a protecting group, and then splitting off

the protecting group and recovering the compounds obtained in free form or in the form of their acid addition salts or quaternary salts.

10. A compound of the formula II as defined in claim 9.

**Claims for the Contracting State:** AT

1. Process for preparing N-acyl-14-O-[(1-amino-2-methylpropan-2-yl)thioacetyl]mutilin- and -19,20-dihydromutilin derivatives, their acid addition salts and quaternary salts, characterised by acylating a compound of formula II

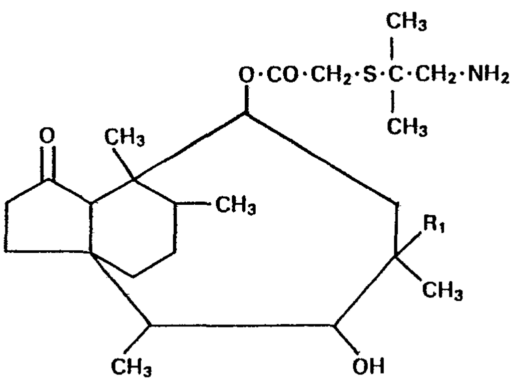

II

wherein $R_1$ stands for ethyl or vinyl, and recovering the compounds obtained in free form or in the form of their acid addition or quaternary salts.

2. Process for preparing pleuromutilin derivatives of formula I

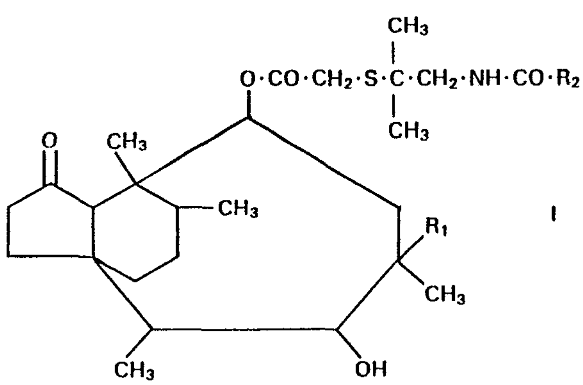

I

wherein $R_1$ stands for ethyl or vinyl and $R_2$ stands for an aminoalkyl group the alkyl moiety of which can be substituted by hydroxy or for a fivemembered saturated heterocycle

and their acid addition or quaternary salts characterised by acylating a compound of formula II

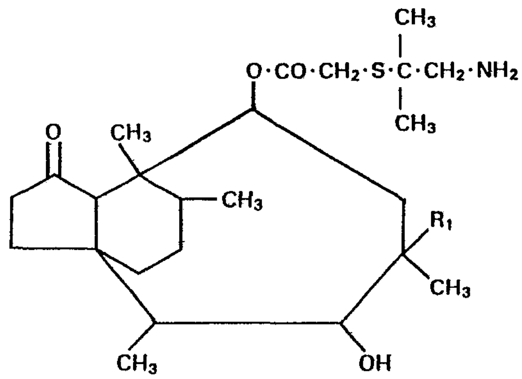

II

with an active ester of the compound of formula III

$$HOOCR_2^{I} \qquad III$$

wherein $R_2^{I}$ has the same meaning as $R_2$ whereby howerer the amino groups contained in $R_2$ are protected by a protecting group, and then splitting off the protecting group and recovering the compounds obtained in free form or in the form of their acid addition salts or quaternary salts.

3. Process according to claim 2 wherein

a) $R_1$ represents vinyl and R represents a fivemembered saturated heterocycle or unsubstituted aminoalkyl and if containing an asymmetric carbon atom is in (D)-Form,

b) $R_1$ represents vinyl and $R_2$ represents amino-hydroxyalkyl.

4. Process according to claim 2 wherein the compound prepared is selected from 14-O-[1-((D)-2-amino-3-methylbutyrylamino)-2-methylpropan-2-yl-thioacetyl]-19,20-dihydromutilin, 14-O-[1-(aminoacetylamino)-2-methylpropan-2-yl-thioacetyl]-mutilin, 14-O-[1-((D)-2-aminopropionylamino)-2-methylpropan-2-yl-thioacetyl]-mutilin, 14-O-[1-(2-amino-4-methylvalerylamino)-2-methylpropan-2-yl-thioacetyl]mutilin, 14-O-[1-((D)-aminopropionylamino)-2-methylpropan-2-yl-thioacetyl]-19,20-dihydromutilin, 14-O-[1-((L)-2-aminopropionylamino)-2-methylpropan-2-yl-thioacetyl]mutilin, 14-O-[1-((L)-thiazolidin-4-yl-carbonylamino)-2-methylpropan-2-yl-thioacetyl]mutilin, 14-O-[1-((L)-pyrrolidin-2-yl-carbonylamino)-2-methylpropan-2-yl-thioacetyl)mutilin, 14-O-[1-(3-aminopropionylamino)-2-methylpropan-2-yl-thioacetyl]-19,20-dihydromutilin, 14-O-[1-((L)-2-amino-3-methylbutyrylamino)-2-methylpropan-2-yl-thioacetyl]mutilin, 14-O-[1-((D)-pyrrolidin-2-yl-carbonylamino)-2-methylpropan-2-yl-thioacetyl]mutilin, 14-O-[1-((L)-2-amino-3-hydroxypropionylamino)-2-methylpropan-2-yl-thioacetyl]mutilin and 14-O-[1-((D)-2-amino-3-methylbutyrylamino)-2-methylpropan-2-yl-thioacetyl]mutilin in the form of the free base, the acid addition salts or the quaternary salts.

5. A compound selected from

a) an N-acyl-14-O-[(1-amino-2-methylpropan-2-yl)thioacetyl]mutilin or -19,20-dihydromutilin.

b) a compound of formula I whereby the substituents are as defined in claim 2.

c) a compound of formula I wherein the substituents are as defined in claim 3.

d) one of the compound given in claim 4.

6. A compound according to claim 5 in the form of the free base, of an acid addition salt or a quaternary salt.

7. A chemotherapeutic composition comprising a compound according to claim 5 in the form of the free base or of a chemotherapeutically acceptable acid addition salt or quaternary salt together with a chemotherapeutically acceptable diluent or carrier or a feed or drinkwater additive composition comprising a compound according to claim 5, in the form of the free base or of a physiologically acceptable acid addition salt or quaternary salt together with a physiologically acceptable diluent or carrier.

8. A compound according to claim 5, a) in the form of the free base or of a chemotherapeutically acceptable acid addition salt or quaternary salt, for use as a chemotherapeutic agent or b) in the form of the free base or of a physiologically acceptable acid addition salt or quaternary salt for use as a veterinary agent.

9. The use of a compound according to claim 5 for preparing agents against bacteria and obligatory anaerobes and for controlling microorganism infections and for promoting growth in domestic animals.

10. A composition according to claim 7 or the use according to claim 8 or 9 wherein the compounds according to claim 5 are prepared according to claim 1.

**Revendications pour les Etats contractants:**
BE, CH, DE, FR, GB, IT, LI, LU, NL, SE.

1. Une N-acyl-14-O-[(1-amino-e-méthylpropane-2-yl)thioacétyl]mutiline ou 19,20-dihydromutiline.

2. Dérivés de pleuromutiline de formule

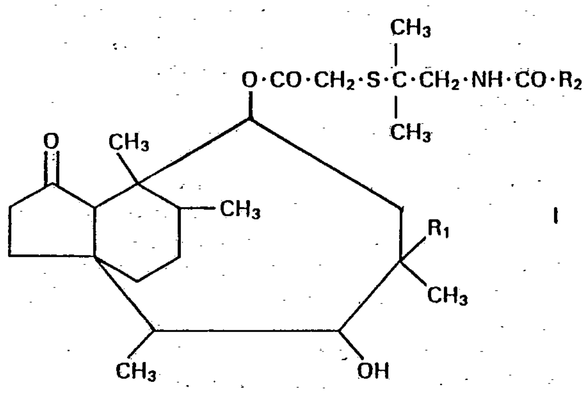

I

dans laquelle $R_1$ représente un groupe éthyle ou vinyle et $R_2$ représente un groupe aminoalkyle dont la partie alkyle peut être substituée par un groupe hydroxy, ou un hétérocycle saturé à 5 chaînons, et leurs sels d'addition d'acides et leurs sels quaternaires.

3. Un composé selon la revendication 2, dans lequel:

a) $R_1$ signifie un groupe vinyle et $R_2$ un hétérocycle saturé à 5 chaînons ou un groupe aminoalkyle non substitué, et lui, lorsqu'il contient un atome de carbone asymétrique, se présente sous la forme D, ou

b) $R_1$ signifie un groupe vinyle et $R_2$ un groupe aminohydroxyalkyle.

4. Un composé choisi parmi la 14-O-[1-((D)-2-amino-3-méthylbutyrylamino)-2-méthylpropane-2-yl-thioacétyl]-19,20-dihydromutiline, la 14-O-[1-(aminoacétylamino)-2-méthylpropane-2-yl-thioacétyl]mutiline, la 14-O-[1-((D)-2-aminopropionylamino)-2-méthylpropane-2-yl-thioacétyl]mutiline, la 14-O-[1-(2-amino-4-méthylvalérylamino)-2-méthylpropane-2-yl-thioacétyl]mutiline, la 14-O-[1-((D)-2-aminopropionylamino)2-méthylpropane-2-yl-thioacétyl]-19,20-dihydromutiline, la 14-O-[1-((L)-2-aminopropionylamino)-2-méthylpropane-2-yl-thioacétyl]mutiline, la 14-O-[1-((L)-thiazolidine-4-yl-carbonylamino)-2-méthylpropane-2-yl-thioacétyl]-mutiline, la 14-O-[1-((L)-pyrrolidine-2-yl-carbonylamino)-2-méthylpropane-2-yl-thioacétyl)mutiline, la 14-O-[1-(3-aminopropionylamino)-2-méthylpropane-2-yl-thioacétyl]-19,20-dihydromutiline, la 14-O-[1-((L)-2-amino-3-méthylbutyrylamino)-2-méthylpropane-2-yl-thioacétyl]mutiline, la 14-O-[1-((D)-pyrrolidine-2-yl-carbonylamino)-2-méthylpropane-2-yl-thioacétyl]mutiline et la 14-O-[1-((L)-2-aminohydroxypropionylamino)-2-méthylpropane-2-yl-thioacétyl]mutiline, sous forme de bases libres, de sels d'addition d'acides ou de sels quaternaires.

5. La 14-O-[1-((D)-2-amino-3-méthylbutyrylamino)-2-méthylpropane-2-yl-thioacétyl]mutiline sous forme de base libre, de sel d'addition d'acide ou de sel quaternaire.

6. Une composition chimiothérapeutique, contenant un composé selon l'une des revendications 1 à 5, sous forme de base libre ou sous forme d'un sel d'addition d'acide ou d'un sel quaternaire acceptable du point de vue chimiothérapeutique, en association avec un diluant ou véhicule acceptable du point de vue chimiothérapeutique, ou bien une composition d'additif pour aliment ou eau de boisson pour animaux, contenant un composé selon l'une des revendications 1 à 5, sous forme de base libre ou sous forme d'un sel d'addition d'acide ou d'un sel quaternaire physiologiquement acceptable, en association avec un diluant ou véhicule physiologiquement acceptable.

7. Un composé selon l'une des revendications 1 à 5, a) sous forme de base libre ou sous forme d'un sel d'addition d'acide ou d'un sel quaternaire acceptable du point de vue chimiothérapeutique, pour l'utilisation comme agent chimiothérapeutique ou bien b) sous forme de base libre ou sous forme d'un sel d'addition d'acide ou d'un sel quaternaire physiologiquement acceptable, pour l'utilisation comme produit vétérinaire.

8. L'utilisation d'un composé selon l'une des revendications 1 à 5 pour la préparation d'agents agissant contre les bactéries et les anaérobes stricts, et pour combattre les infections provoquées par les microorganismes ainsi que pour favoriser la croissance des animaux domestiques et des animaux utiles.

9. Procédé de préparation de dérivés de N-acyl-14-O-[(1-amino-2-méthylpropane-2-yl)thioacétyl]-mutiline et 19,20-dihydromutiline, en particulier ceux de formule I tels que définis à la revendication 2, de leurs sels d'addition d'acides et de leurs sels quaternaires, caractérisé en ce qu'on acyle un composé de formule

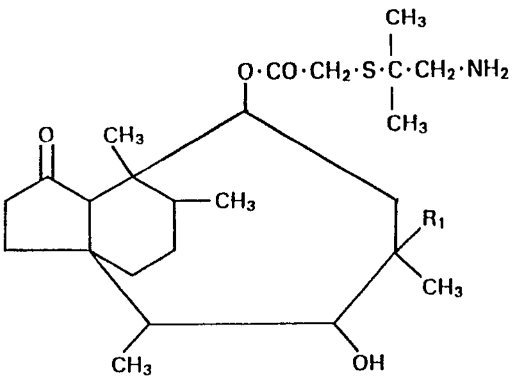

II

dans laquelle $R_1$ représente un groupe éthyle ou vinyle, en particulier avec un ester activé du composé de formule

HOOC-$R_2^I$ III

dans laquelle $R_2^I$ a la même signification que $R_2$, les groupes amino qui se trouvent dans $R_2$ étant cependant protégés par un groupe protecteur, et on élimine ensuite le groupe protecteur et on récupère les composés obtenus sous forme libre ou sous forme de leurs sels d'addition d'acides ou de leurs sels quaternaires.

10. Un composé de formule II tel que défini à la revendication 9.

**Revendications pour l'Etat contractant:** AT

1. Procédé de préparation de dérivés de N-acyl-14-O-[(1-amino-2-méthylpropane-2-yl)thioacétyl]-mutiline et 19,20-dihydromutiline, de leurs sels d'addition d'acides et de leurs sels quaternaires, caractérisé en ce qu'on acyle un composé de formule II

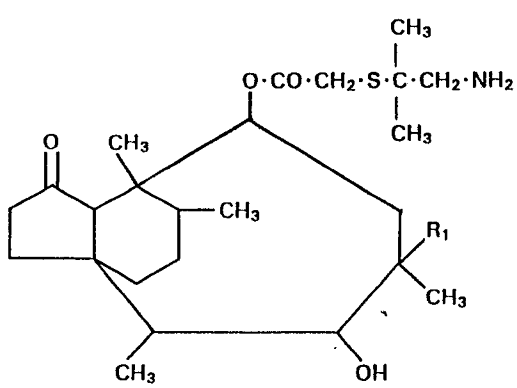

II

dans laquelle $R_1$ représente un groupe éthyle ou vinyle, et on récupère les composés obtenus sous forme libre ou sous forme de leurs sels d'addition d'acides ou de leurs sels quaternaires.

2. Procédé de préparation de dérivés de pleuromutiline de formule I

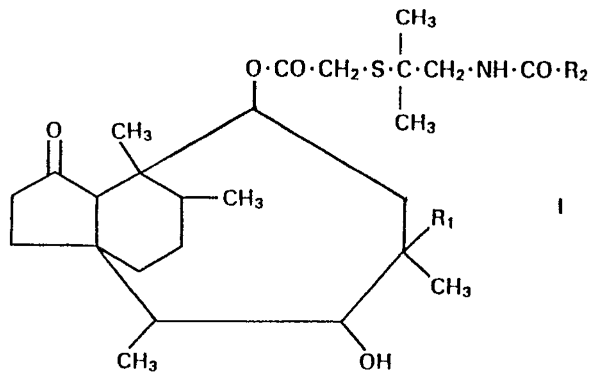

I

dans laquelle $R_1$ représente un groupe éthyle ou vinyle et $R_2$ représente un groupe aminoalkyle dont la partie alkyle peut être substituée par un groupe hydroxy, ou un hétérocycle saturé à 5 chaînons, ainsi que de leurs sels d'addition d'acides et de leurs sels quaternaires, caractérisé en ce qu'on acyle un composé de formule II

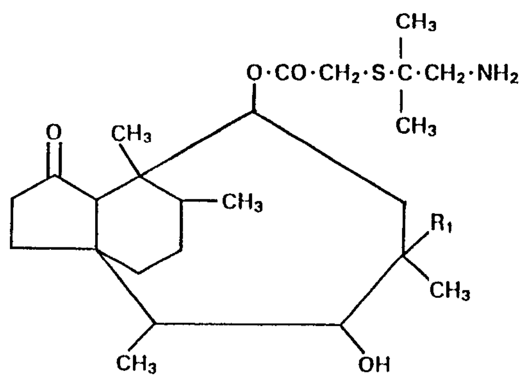

II

avec un ester activé du composé de formule III

HOOC-$R_2^I$ III

dans laquelle $R_2^I$ a la même signification que $R_2$, les groupes amino qui se trouvent dans $R_2$ étant cependant protégés par un groupe protecteur, et on élimine ensuite le groupe protecteur et on récupère les composés obtenus sous forme libre ou sous forme de leurs sels d'addition d'acides ou de leurs sels quaternaires.

3. Procédé selon la revendication 2, dans lequel a) $R_1$ signifie un groupe vinyle et $R_2$ un hétérocycle saturé à 5 chaînons ou un groupe aminoalkyle non substitué, et qui, lorsqu'il contient un atome de carbone asymétrique, se présente sous la forme D, ou b) $R_1$ signifie un groupe vinyle et $R_2$ un groupe aminohydroxyalkyle.

4. Procédé selon la revendication 2, dans lequel le composé préparé est choisi parmi la 14-O-[1-((D)-2-amino-3-méthylbutyrylamino)-2-méthylpropane-2-yl-thioacétyl]-19,20-dihydromutiline, la 14-O-[1-(aminoacétylamino)-2-méthylpropane-2-yl-thioacétyl]mutiline, la 14-O-[1-((D)-2-aminopropionylamino)-2-méthylpropane-2-yl-thioacétyl]mutiline, la 14-O-[1-(2-amino-4-methylvalérylamino)-2-méthylpropane-2-yl-thioacétyl]mutiline, la 14-O-[1-((D)-aminopropionylamino)-2-méthylpropane-2-yl-thioacétyl]-19,20-dihydromutiline, la 14-O-[1-((L)-2-aminopropionylamino)-2-méthylpropane-2-yl-thioacétyl]mutiline, la 14-O-[1-((L)-thiazolidine-4-yl-carbonylamino)-2-méthylpropane-2-yl-thioacétyl]mutiline, la 14-O-[1-((L)-pyrrolidine-2-yl-carbonylamino)-2-méthylpropane-2-yl-thioacétyl)mutiline, la 14-O-[1-(3-aminopropionylamino)-2-méthylpropane-2-yl-thioacétyl]-19,20-dihydromutiline, la 14-O-[1-((L)-2-amino-3-méthylbutyrylamino)-2-méthylpropane-2-yl-thioacétyl]mutiline, la 14-O-[1-((D)-pyrrolidine-2-yl-carbonylamino)-2-méthylpropane-2-yl-thioacétyl]mutiline, la 14-O-[1-((L)-2-amino-3-hydroxypropionylamino)-2-méthylpropane-2-yl-thioacétyl]mutiline et la 14-O-[1-((D)-2-amino-3-méthylbutyrylamino)-2-méthylpropane-2-yl-thioacétyl]mutiline, chacun sous forme de base libre ou sous forme de sels d'addition d'acides ou de sels quaternaires.

5. Un composé choisi parmi

a) une N-acyl-14-O-[(1-amino-2-méthylpropane-2-yl)thioacétyl]mutiline ou 19,20-dihydromutiline;

b) un composé de formule I, les substituants étant tels que définis à la revendication 2;

c) un composé de formule I dans laquelle les substituants sont tels que définis à la revendication 3;

d) un des composés indiqués à la revendication 4.

6. Un composé selon la revendication 5 sous forme de base libre, de sel d'addition d'acide ou de sel quaternaire.

7. Une composition chimiothérapeutique, contenant un composé selon la revendication 5 sous forme de base libre ou sous forme d'un sel d'addition d'acide ou d'un sel quaternaire acceptable du point de vue chimiothérapeutique, en association avec un diluant ou véhicule acceptable du point de vue chimiothérapeutique, ou bien une composition d'additif pour aliment ou eau de boisson pour animaux, contenant un composé selon la revendication 5 sous forme de base libre ou sous forme d'un sel d'addition d'acide ou d'un sel quaternaire physiologiquement acceptable, en association avec un diluant ou véhicule physiologiquement acceptable.

8. Un composé selon la revendication 5, a) sous forme de base libre ou sous forme d'un sel d'addition d'acide ou d'un sel quaternaire acceptable du point de vue chimiothérapeutique, pour l'utilisation comme agent chimiothérapeutique ou bien b) sous forme de base libre ou sous forme d'un sel d'addition d'acide ou d'un sel quaternaire physiologiquement acceptable, pour l'utilisation comme produit vétérinaire.

9. L'utilisation d'un composé selon la revendication 5 pour la préparation d'agents agissant contre les bactéries et les anaérobes stricts, et pour combattre les infections provoquées par les micro-organismes ainsi que pour favoriser la croissance des animaux domestiques et des animaux utiles.

10. Une composition selon la revendication 7 ou l'utilisation selon la revendication 8 ou 9, où les composés selon la revendication 5 sont préparés selon la revendication 1.